(19)

**Europäisches Patentamt**
**European Patent Office**
**Office européen des brevets**

(11) **EP 4 355 473 B1**

(12) **EUROPEAN PATENT SPECIFICATION**

(45) Date of publication and mention
of the grant of the patent:
**09.07.2025 Bulletin 2025/28**

(21) Application number: **22730506.7**

(22) Date of filing: **25.05.2022**

(51) International Patent Classification (IPC):
**B01J 19/00** *(2006.01)* **B01J 19/24** *(2006.01)*
**C07C 51/25** *(2006.01)* **B01J 8/06** *(2006.01)*

(52) Cooperative Patent Classification (CPC):
(C-Sets available)
**B01J 19/0013; B01J 8/067; B01J 19/002;**
**B01J 19/2445; C07C 51/252;** B01J 2208/00256;
B01J 2208/00716; B01J 2208/00769;
B01J 2219/00036; B01J 2219/00247;
B01J 2219/2401 (Cont.)

(86) International application number:
**PCT/EP2022/064316**

(87) International publication number:
**WO 2022/263139 (22.12.2022 Gazette 2022/51)**

(54) **PROCESS FOR SHUTTING-DOWN AND HEATING UP A TUBULAR REACTOR FOR A CATALYTIC GAS PHASE REACTION**

VERFAHREN ZUM ABSCHALTEN UND AUFHEIZEN EINES ROHRREAKTORS FÜR EINE KATALYTISCHE GASPHASENREAKTION

PROCESSUS D'ARRÊT ET DE CHAUFFAGE D'UN RÉACTEUR TUBULAIRE POUR UNE RÉACTION CATALYTIQUE EN PHASE GAZEUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.06.2021 EP 21179892**

(43) Date of publication of application:
**24.04.2024 Bulletin 2024/17**

(73) Proprietor: **BASF SE**
**67056 Ludwigshafen am Rhein (DE)**

(72) Inventors:
• **HAMMON, Ulrich**
**68163 Mannheim (DE)**

• **RAITH, Christian**
**67056 Ludwigshafen am Rhein (DE)**
• **BASSLER, Hans-Juergen**
**67056 Ludwigshafen am Rhein (DE)**

(74) Representative: **BASF IP Association**
**BASF SE**
**Global Intellectual Property**
**GBI, Building Z078**
**Carl-Bosch-Strasse 38**
**67056 Ludwigshafen (DE)**

(56) References cited:
EP-A2- 1 882 518     US-A1- 2001 024 630
US-A1- 2005 101 803     US-A1- 2008 269 521

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 51/252, C07C 57/04**

## Description

The present invention relates to a process for shutting-down a tubular reactor for a catalytic gas phase reaction from a reaction temperature, wherein the tubular reactor comprises a plurality of vertically arranged reaction tubes, an upper tube sheet and a lower tube sheet which each are connected to upper ends and lower ends of the reaction tubes in a gas-tight manner, and a reactor shell which encloses the plurality of reaction tubes forming a liquid-tight heat transfer space, wherein, in operation mode, a substantially anhydrous liquefied salt melt is circulated in the heat transfer space. The invention further relates to a process for heating the tubular reactor from the temperature below the solidification temperature of the liquefied salt melt or lower to the reaction temperature after the cooling step.

Catalytic gas-phase reactions in chemical industry, such as oxidation, hydrogenation, dehydrogenation, nitration, alkylation etc., are usually performed using tubular reactors with isothermal fixed beds. Such reactions performed in tubular reactors may be either endothermic or exothermic. The fixed bed, usually a granular catalyst, is located in reaction tubes of the tubular reactor. Nowadays, commonly used tubular reactors may have at least 5,000 and up to 45,000 reaction tubes. This plurality of reaction tubes is referred to as reaction tube bundle, which is generally annular and arranged vertically and surrounded by a reactor shell. Both ends of said reaction tube bundle are sealed in tube sheets. The feed gas mixture is usually introduced at the top part of the tubular reactor via a hood and fed to the reaction tubes via the tube sheet. A resulting product gas mixture is discharged at the bottom part of the tubular reactor via the opposite tube sheet and hood. Alternatively, the feed gas is introduced at the bottom part of the tubular reactor and leaves the tubular reactor via the upper tube sheet and hood.

Stable reaction conditions, i.e. carrying out the reaction at uniform process heat dissipation and good heat exchange, are usually achieved by circulating a heat transfer medium through the space between the reaction tube bundle and the reactor shell at a constant temperature using a pump. Doing so enables cooling or heating of the reaction tubes, followed by cooling or heating of the heat transfer medium in a heat exchanger. The heat transfer medium usually flows meandering through the reactor tube bundle. In order to obtain similar reaction conditions for all reaction tubes, the heat transfer medium should be distributed as evenly as possible. For example, salt mixtures such as eutectic salt mixtures, e.g. comprising potassium nitrate and sodium nitrite, may be used as heat transfer medium.

A major challenge of performing a reaction in a tubular reactor as described above relies in maintenance of the reactor, e.g. for catalyst replacements. Such catalyst replacement requires a complete shut-down and cooling down of the tubular reactor, and re-heating after maintenance is finished, in order to be able to access the fixed beds containing the catalyst. In other words, such shut-downs of the reaction, respectively shut-downs of the tubular reactor, may also be referred to as stand-by. Usually, the heat transfer medium, i.e. the liquefied salt melt, is cooled to a temperature below the solidification temperature of the liquefied salt melt, e.g. to room temperature, resulting in solidification of the salt melt. Consequently, re-starting the reaction, i.e. heating up, requires re-heating of the solidified salt melt above its melting point, usually to at least 150 °C which is both complicated and time- and resource-consuming.

EP 1 882 518 B1 describes a process of varying the temperature of a tube bundle reactor for catalytic gas phase reactions upon start up and shut-down. The tube bundle reactor comprises a major reactor portion which includes a bundle of vertically disposed reactor tubes, upper and lower tube sheets tightly connected to the upper and lower ends, respectively, of the reactor tubes, and a reactor shell enclosing the tube bundle. A heat transfer medium having a melting temperature in the range of from 100 to 450 °C flows around the outer surfaces of the reactor tubes during normal operation and being circulated in at least one circuit through the major reactor portion. The process comprises the steps of (a) varying the heat transfer medium temperature by means of a heat exchanger during circulation of the heat transfer medium; and (b) passing a temperature gas though the reactor tubes at least when the heat transfer medium is not yet or no longer circulated. The process is characterized in that the temperature of the temperature gas, when entering the reactor tubes, is limited upwards during start up and downwards during shut-down and/or the volume flow of the temperature gas is limited upwards such that, for any time period which begins with the first introduction of the temperature gas, the time average rate of change of the temperature of the temperature gas, when exiting the reactor tubes, does not exceed 30 °C/h. However, said process has the disadvantage that the time average rate of change of the temperature of the temperature gas is limited to be 30 °C/h at maximum. Thus, such start up and shut-down of the tube bundle reactor is a time- and resource-consuming process resulting in a process of reduced efficiency as a long service life due to slow cooling and heating up is required.

It is therefore an object of the present invention to provide a process for shutting-down and heating up a tubular reactor in a way that a simpler and faster process is obtained which requires less constructional effort, causes less maintenance and is less cost-intensive.

According to the present invention, this object is achieved by a process having the features of claim 1 and/or a process having the features of claim 11. Advantageous configurations and developments are apparent from the dependent claims.

Accordingly, the invention relates to a process for shutting-down a tubular reactor for a catalytic gas phase reaction from a reaction temperature, wherein the tubular reactor comprises a plurality of vertically arranged reaction tubes, an upper tube sheet and a lower tube sheet which each are connected to upper ends and lower ends of the reaction tubes in a gas-tight manner, and a reactor shell which encloses the plurality of reaction tubes forming a liquid-tight heat transfer space, wherein, in operation mode, a substantially anhydrous liquefied salt melt is circulated in the heat transfer space. The process is characterized in that water is added to the substantially anhydrous liquefied salt melt, obtaining a water-salt mixture, while cooling the tubular reactor to a temperature below the solidification temperature of the substantially anhydrous liquefied salt melt, such that the water-salt mixture is kept in a liquefied state during the whole cooling step of the tubular reactor.

The process of the present invention is performed in a tubular reactor. The characteristic design of such a tubular reactor is known per se to the skilled person. For the sake of completeness, the components of such a tubular reactor are summarized in the following: The tubular reactor is limited by a "reactor shell", which suitably is a cylindrical body, and, on the upper end and the lower end of said reactor shell, by an "upper hood" and a "lower hood", wherein the upper hood and the lower hood are connected to the reactor shell in a gas-tight manner. Suitably, the inner diameter of the upper hood and the lower hood are equal to the inner diameter of the reactor shell. Inside the tubular reactor, a plurality of vertically arranged "reaction tubes" is present in such a way that the reactor shell encloses the plurality of reaction tubes. Generally, the term "plurality" of reaction tubes denotes a huge number of reaction tubes present inside the tubular reactor, e.g. at least 5,000 and up to 45,000 reaction tubes. Suitably, said reaction tubes have an inner diameter of 20 to 30 mm, a length of 2 to 4 m, and a wall thickness of 1 to 3 mm. The upper ends of the reaction tubes are connected to an "upper tube sheet" and the lower ends of the reaction tubes are connected to a "lower tube sheet", each in a gas-tight manner. In other words, both ends of the reaction tubes are sealed in the tube sheets. Thus, a gas-tight "reaction space" is formed by the space between the upper hood and the upper tube sheet, inside the reaction tubes, and the space between the lower tube sheet and the lower hood. In said reaction space, the feed gas mixture is introduced into the tubular reactor, subjected to a chemical reaction envisaged in the tubular reactor inside the reaction tubes, and removed from the tubular reactor afterwards.

A second space, also referred to as the "heat transfer space", is formed inside the tubular reactor between the upper tube sheet, the lower tube sheet, and outside the reaction tubes. Said heat transfer space is suitably designed in a liquid-tight manner. In other words, the heat transfer space is a separate space located outside of the reaction space and inside of the reactor shell.

As mentioned above, the purpose of the reaction tubes is to enable performing a reaction envisaged in the tubular reactor, e.g. a "catalytic gas phase reaction" such as a catalytic gas phase oxidation inside the reaction tubes. Thus, inside the vertically arranged reaction tubes of the tubular reactor, a granular catalyst is present, e.g. in the form of a fixed bed, which is suitable for performing the reaction envisaged in the tubular reactor.

During "operation mode" of the tubular reactor, the reaction envisaged in the tubular reactor is performed. The reaction is performed at a reaction temperature, which is generally an elevated temperature. In the event that the reaction envisaged in the tubular reactor is a catalytic gas phase oxidation, e.g. a catalytic gas phase oxidation of propylene via acrolein yielding acrylic acid in two consecutive reactors, the reaction temperature for the first stage for the conversion of propylene to acrolein is in the range of 300 to 400 °C, preferably 320 to 380 °C, and the reaction temperature for the second stage for the conversion of acrolein to acrylic acid is in the range of 240 to 310 °C, preferably 260 to 300 °C.

Suitably, for performing said reaction during operation mode, a feed gas mixture, e.g. comprising propylene or acrolein, is introduced into the inlet part of the tubular reactor, i.e. at the upper hood. Subsequently, the feed gas mixture enters the reaction tubes via the upper tube sheet at the upper ends of the reaction tubes and flows through the reaction tubes in direction from the upper ends of the reaction tubes towards the lower ends of the reaction tubes, i.e. in direction to the lower tube sheet. Thus, a product gas mixture, e.g. comprising acrylic acid, leaves the reaction tubes at their lower ends via the lower tube sheet and may then be discharged at the bottom part of the tubular reactor via the lower hood.

Generally, the elevated temperature levels which are required for performing the reaction in the tubular reactor are provided by a heat transfer medium. It is very important to provide stable reaction conditions, i.e. to carry out the reaction at constant temperature levels. In order to provide such stable reaction conditions, the heat transfer medium is suitably circulated through the heat transfer space between the reaction tubes and the reactor shell in an overall longitudinal direction at the intended temperature using a pump. Doing so enables cooling or heating of the reaction tubes, followed by cooling or heating of the heat transfer medium in a heat exchanger, e.g. an externally arranged heat exchanger. Generally, for this purpose, the reaction tubes are arranged in the tubular reactor such that they are equidistant from each other. Suitably, the distance between the centric inner axes of two adjacent reaction tubes is in the range of 35 to 45 mm.

For example, suitable heat transfer media are fluid heat transfer media such as "substantially anhydrous liquefied salt melts" or low-melting metals such as sodium, mercury or alloys of different metals. Suitable substantially anhydrous liquefied salt melts may be eutectic salt mixtures, e.g. comprising sodium nitrate, potassium nitrate, sodium nitrite and/or potassium nitrite.

Generally, all above-described components of the tubular reactor are manufactured of a material which is suitable for performing the reaction envisaged inside the reactor, e.g. the catalytic gas phase oxidation such as an oxidation reaction of propylene via acrolein to acrylic acid.

Herein, the terms "shutting-down" or "shut-down" of the tubular reactor refer to a state of the reactor where the operation mode is interrupted. For example, a shut-down is performed in order to carry out maintenance work. A common example of recurring necessary maintenance work is catalyst replacement after the catalyst located inside the reaction tubes has exceeded its lifetime. In such an event, the tubular reactor needs to be opened for being able to remove the spent catalyst and refill the reaction tubes with new catalyst. The tubular reactor may only be opened after interruption of the reaction and cooling of the tubular reactor to a temperature near room temperature, e.g. via removing heat by the heat transfer medium with the aid of a heat exchanger. As already described above, up to now, such cooling step of a tubular reactor to room temperature for shut-downs are the bottleneck of maintenance processes in terms of time and resource expenditure. This is because the liquefied salt melt solidifies upon cooling to room temperature. This results in limitations regarding cooling and heating rates of the heat transfer medium in order to avoid tensions inside the reactor which could result in damages such as cracks. Furthermore, huge amounts of energy are required to slowly re-heat the solidified salt melt to its melting temperature.

The inventors have now surprisingly found that a process according to the present invention overcomes these disadvantages: At the beginning of the shut-down, water is added to the substantially anhydrous liquefied salt melt via a water tube, i.e. to the heat transfer medium, thus obtaining a water-salt mixture. Said water-salt mixture may then be cooled down, e.g. to room temperature, without limitations in cooling rates and later be re-heated up to the reaction temperature, also without limitations in heating rates. In other words, the tubular reactor may be cooled to a temperature below the solidification temperature of the substantially anhydrous liquefied salt melt, e.g. to room temperature, after addition of water to the liquefied salt melt, very quickly. This results in a shut-down process which is a simpler and faster process causing less maintenance. Furthermore, the water-salt mixture exhibits a viscosity similar to the viscosity of water, i.e. a lower viscosity in comparison to the viscosity of the liquefied salt melt. This is advantageous in view of easier pumpability of the water-salt mixture; thus, less strong pumps are required and less energy is consumed by said less strong pumps resulting in a more efficient process. Another advantage of the addition of water relies in the high heat of solution of water. This means that upon addition of water to the liquefied salt melt, at least a part of the heat contained in the heat transfer medium, i.e. in the liquefied salt melt, is absorbed by the water. This advantageously simplifies and accelerates the cooling step and results in a process which is less time- and resource-intensive.

As described above, conventional shut-down processes usually keep the liquefied salt melt itself in its liquefied state. Thus, the liquefied salt melt needs to be stored outside of the tubular reactor, e.g. in a heated reservoir. This is because the tubular reactor needs to be cooled below the solidification temperature of the liquefied salt melt and in the event that the liquefied salt melt would stay inside the tubular reactor, it would solidify. This approach requires huge constructional effort in the form of installation of heated reservoirs providing large volumes and heated tubes for transferring the liquefied salt melt from the tubular reactor into the heated reservoir. All of this is very cost-intensive in both installations and during operation. In the process according to the present invention, after addition of water to the liquefied salt melt, the water-salt mixture is kept in a liquefied state during the whole cooling step of the tubular reactor, even at room temperature. In comparison to the above-described conventional shut-down process, this approach requires by far less constructional effort as no heated reservoirs and/or tubes are necessary. Also, most of the water-salt mixture may be kept inside the reactor itself during shut-down. Furthermore, in the conventional shut-down process as described above, the liquefied salt melt stored in the heated reservoir needs to be heated during the whole maintenance process in order to keep the liquefied salt melt in its liquefied state. In comparison thereto, this step is omitted in the process according to the present invention as the water-salt mixture does not need to be heated as it stays in the liquefied state even at room temperature. This is advantageous in view of less energy requirement, or, in other words, in view of less resource consumption and reduced process costs.

In summary, the process according to the present invention does not require cooling the liquefied salt melt itself to room temperature accompanied by solidification of said salt melt and re-heating it to at least the melting point of the solidified salt melt. Also, the water-salt mixture does not need to be heated during shut-down as it stays in the liquefied state even at room temperature. Thus, a simpler and faster shut-down process causing less maintenance is obtained which is especially advantageous in view of less energy requirement, or, in other words, in view of less resource consumption and reduced process costs. The water-salt mixture has a lower viscosity in comparison to the viscosity of the liquefied salt melt; this is advantageous in view of pumpability of the water-salt mixture resulting in a simpler process. In contrast to the conventional shut-down process which is limited in view of the cooling rate of the heat transfer medium, the process according to the present invention does not have such limitations regarding the cooling rate. Another advantage of the process according to present invention relies in the high heat of solution of water; upon addition of water to the liquefied salt melt, at least a part of the heat contained in the liquefied salt melt is absorbed by the water. This advantageously results in a simplification and acceleration of the shut-down process resulting in a less time- and resource-intensive process. Further advantageously, less constructional effort such as installation of heated reservoirs for storing the liquefied salt melt is necessary as most of the water-salt mixture may be kept inside the reactor itself during shut-down and thus, no additional investment is necessary for carrying out the process according to the present invention.

In an embodiment, the water-salt mixture is cooled to a temperature in the range of 80 to 10 °C, preferably 60 to 20 °C, more preferably to room temperature. This results in an even more advantageous shut-down process due to an easier and less dangerous maintenance process during shut-down at such reduced temperature levels.

In an embodiment, the water is added to obtain a weight ratio of water to liquefied salt melt of 80 : 20 to 40 : 60, preferably 60 : 40 to 40 : 60. In this context, the term "weight ratio" denotes the ratio of the weight of water to the weight of the liquefied salt melt. This results in an even more advantageous shut-down process because the so obtained water-salt mixture exhibiting said preferred water content provides especially preferred conditions regarding heat of solution of water and viscosity of the water-salt mixture. Furthermore, the water-salt mixture having the above ratio of water to liquefied salt melt is a solution during the whole shut-down process, i.e. the precipitation of solid salt crystals in the water-salt mixture is avoided. This results in a simplification and acceleration of the cooling step and a less time- and resource-intensive process causing less maintenance.

In an embodiment, the liquefied salt melt has a melting temperate in the range of 100 to 450 °C, preferably 140 to 155 °C. This results in an even more advantageous shut-down process having the advantages as described above.

In an embodiment, the liquefied salt melt has a solubility such that 1 kg of liquefied salt melt is soluble in less than 6 L, preferably less than 3 L of water, at 20 °C. This results in an even more advantageous shut-down process as a sufficient miscibility of the liquefied salt melt and water is ensured, thus ensuring that a process having the advantages as described above is obtained.

In an embodiment, the liquefied salt melt is a eutectic mixture comprising nitrate moieties, preferably a mixture of at least two salts selected from alkali nitrates, alkali nitrites and alkali carbonates, preferably a mixture of two or three of the salts potassium nitrate, sodium nitrate and sodium nitrite.

Generally and in the context of the present patent application, the term "eutectic mixture" denotes a homogeneous mixture of substances which melts or solidifies at a single temperature which is lower than the melting point of any of the constituents.

The use of an above-described eutectic mixture as liquefied salt melt results in an even more advantageous shut-down process having the advantages as described above.

In an embodiment, the liquefied salt melt consists of a mixture of the salts potassium nitrate and sodium nitrite in a weight ratio of potassium nitrate to sodium nitrite of 45 : 65 to 65 : 45, preferably 50 : 50 to 60 : 40. In this context, the term "weight ratio" denotes the ratio of the weight of potassium nitrate to the weight of sodium nitrite. The use of such a mixture as liquefied salt melt results in an even more advantageous shut-down process having the advantages as described above.

In an embodiment, at least a part of the water-salt mixture is kept inside the tubular reactor. In other words, the remainder of the water-salt mixture may be stored outside the tubular reactor. Still, this is advantageous because there is no need to provide a reservoir which is large enough for storing the whole volume of water-salt mixture, but solely the part of the water-salt mixture which is stored outside the tubular reactor. The storage volume can be provided via temporally facilities like containers as the salt water mixture requires no additional requirements for storage like heating.

In the context of the present application, the term "reservoir" may be referred to as, e.g., an already existing reservoir, e.g. a salt melt reservoir.

This results in an even more advantageous shut-down process as less constructional effort is required; in the event that an already existing reservoir can be used, the process is simpler, faster and less cost-intensive.

In an embodiment, the water-salt mixture which is kept inside the tubular reactor is continuously circulated through the tubular reactor. Doing so enables to more efficiently dissipate the heat during cooling. Thus, there are no limitations regarding the heating rate and/or the cooling rate of the water-salt mixture, and of the tubular reactor itself. This results in an even more advantageous shut-down process which is simpler and faster and which causes less maintenance, thus resulting in a less time- and resource-intensive process.

In an embodiment, at least a part of the water-salt mixture is stored outside the tubular reactor, preferably the water-salt mixture is introduced into a non-heated reservoir via a drain tube.

Herein, the term "drain tube" refers to a non-heated hose which allows for discharging at least a part of the water-salt mixture from the tubular reactor into, for example, a non-heated reservoir. The addition of water to the liquefied salt melt results in an increase of volume of the water-salt mixture in comparison to the liquefied salt melt. Thus, it may be necessary to discharge at least a part of the water-salt mixture in the event that the total volume of water-salt mixture is larger than the volume available inside the tubular reactor. Thus, the drain tube is suitably arranged at a position of the tubular reactor such that a suitable volume of water-salt mixture may be discharged to external, e.g. to the above-described non-heated reservoir.

This is advantageous because there is no need to provide a reservoir which is large enough for storing the whole volume of water-salt mixture but solely the part of the water-salt mixture which is stored outside the tubular reactor. This results in an even more advantageous shut-down process as less constructional effort is required for providing only smaller reservoirs in comparison to conventional shut-down processes. Furthermore, said reservoirs do not need to be heatable. Thus, the process is simpler, faster and less cost-intensive.

The present invention further relates to a process for heating up the tubular reactor from the temperature below the solidification temperature of the liquefied salt melt or lower to the reaction temperature after the cooling step as described above, wherein at least a part of the water is boiled out from the water-salt mixture at a temperature above the solidification temperature of the liquefied salt melt by supplying heat via a heat exchanger obtaining a water steam and resulting in a volume reduction of the water-salt mixture. The heat may be supplied by using electrical energy and/or steam and/or a tempering gas which is sent through heat exchanger tubes during the heating up obtaining a steam vent out of a vent line of a salt circulation system and resulting in a volume reduction of the water-salt mixture.

Herein, the term "heating up" of the tubular reactor overall refers to a reversal of the cooling step. In other words, during heating up, the tubular reactor is transferred from stand-by after cooling back to operation mode so that the operation mode is no longer interrupted. For example, the heating up step may be carried out after maintenance work, e.g. catalyst replacement, is finished and the operation of the reactor may be resumed.

For "reversing" the above-described cooling step, it is necessary to a) increase the temperature above the solidification temperature of the liquefied salt melt, for finally regaining the reaction temperature, and b) to reduce the volume of the water-salt mixture, i.e. to remove water from the water-salt mixture in order to return to the original composition of the heat transfer medium of before the shut-down, i.e. to the substantially anhydrous liquefied salt melt.

Herein, steps a) and b) are correlated. Performing the temperature increasing step a) automatically results in a removal of water, step b), by boiling out the water from the water-salt mixture after reaching a temperature of at least 100 °C, as described above. Consequently, the boiling out of water results in a volume reduction of the water-salt mixture.

Suitably, the temperature is increased by supplying heat via the heat exchanger which is already present in the tubular reactor, as described above. The heat exchanger may be operated using a tempering gas. In the context of the present application, the tempering gas may suitably be selected from reaction gas, air, smoke gas, steam, inert gas etc., preferably steam.

The heating up process according to the present invention is especially advantageous due to the following reasons: re-heating a solidified salt melt to at least the melting point of the salt melt is not required as after addition of water during the shut-down process solely the water-salt mixture instead of the liquefied salt melt itself has been cooled. In contrast to the salt melt, the water-salt mixture does not solidify upon cooling. Thus, a simpler and faster shut-down process causing less maintenance is obtained. In contrast to the conventional shut-down process which is limited in view of the heating rate of the heat transfer medium, the process according of the invention does not have such limitations regarding the heating rate. Thus, the process according to the present invention is especially advantageous in view of quickly reaching the reaction conditions. This considerably reduces the time and maintenance requirements of said process compared to conventional processes, e.g. from several days to few hours.

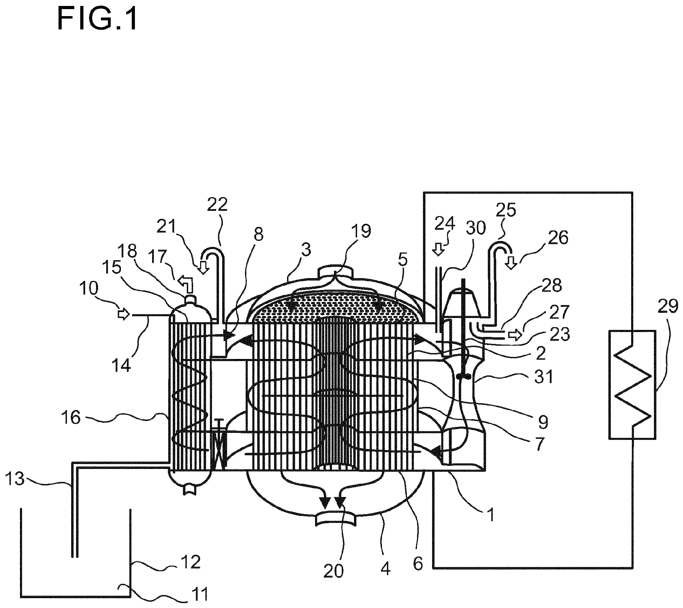

In an embodiment, the water steam is released from the tubular reactor, preferably via the vent of the salt melt circulation system or an emergency release line as described in DE 10 2014 103 691 A1, figures 1 and 2 (reference sings 17, 19, and/or 33).

This results in an even more advantageous shut-down process as on the one hand, such installation ensures that harmful reaction conditions in the form of pressures exceeding the design limits of the reactor may not be reached. On the other hand, the installation of a pressure relief valve is a very easy and inexpensive way of allowing the water to be boiled out of the water-salt-mixture to leave the tubular reactor.

In an embodiment, the volume reduction of the water-salt mixture is compensated by adding at least a part of the water-salt mixture which is stored outside the tubular reactor. Practically, after a certain volume of water has been boiled out of the water-salt mixture, a similar volume of the externally stored water-salt mixture is pumped back into the tubular reactor. Afterwards, another volume of water is boiled out and the preceding steps are repeated. This results in an even more advantageous shut-down process as the externally stored water-salt mixture may be re-used by continuously recycling the externally stored water-salt mixture. Otherwise, new salt(s) would have to be added which is disadvantageous in view of process costs and resource consumption.

In an embodiment, the tubular reactor is cooled down, a catalyst which is present inside the reaction tubes is replaced, and the tubular reactor is then heated up. Such a procedure is generally referred to as "catalyst replacement shut-down". Such a catalyst replacement shut-down becomes necessary when the catalyst located inside the reaction tubes of the tubular reactor has exceeded its lifetime. In this event, the catalyst is no longer active and the intended reaction cannot be carried out. Conventional shut-down processes for catalyst replacement shut-downs have the disadvantages as described in detail above. By carrying out the shut-down process in accordance with the present invention, a more advantageous shut-down process for catalyst replacement is obtained having the advantages as described above.

In an embodiment, the gas phase reaction is selected from oxidation, hydrogenation, dehydrogenation, nitration, and alkylation reactions, preferably an oxidation reaction, in particular an oxidation reaction of propylene via acrolein to acrylic acid. Such processes have a common feature as they are generally carried out in the presence of a suitable catalyst. Thus, they require a catalyst replacement shut-down when the catalyst used has exceeded its lifetime and is thus no longer active for carrying out the intended reaction. Conventional shut-down processes for catalyst replacement shut-downs have the disadvantages as described in detail above. By carrying out the shut-down process in accordance with the present invention, a more advantageous shut-down process for catalyst replacement is obtained having the advantages as described above.

The present invention is described in detail below with reference to the attached figure and examples.

The figure depicts a schematic vertical section of a tubular reactor in a working example of the present invention.

Examples

Two preliminary experiments in laboratory-scale have been performed in order to investigate whether a eutectic salt mixture comprising 60 wt.-% of $KNO_3$ and 40 wt.-% of $NaNO_2$ may be used for cooling a tubular reactor during shut-down. In other words, the goal of these preliminary experiments was to find out whether above eutectic salt mixture may be mixed with water while controlling the water concentration such that the resulting water-salt mixture may be cooled down from an elevated temperature to room temperature without generating any solid salt crystals. The absence of such solid crystals is important. This is because on an industrial scale, the formation of solid crystals must be avoided as re-heating a solidified salt melt requires specific parameters in reaction control. For example, the heating rate of a solidified salt melt is limited as in the event of too fast heating, tensions inside the reactor may occur which could result in damages such as cracks.

Example 1

In the context of example 1, a water-salt mixture consisting of a eutectic salt mixture comprising 60 wt.-% of $KNO_3$ and 40 wt.-% of $NaNO_2$, and 1 wt.-% of water has been prepared. For this water-salt mixture, the above-described elevated temperature has been determined at which the water fraction of said water-salt mixture starts boiling. The temperature has been determined according to the following procedure: A 250 mL three-necked flask equipped with a condenser and a thermometer has been charged with 100 g of a salt mixture comprising 60 wt.-% of $KNO_3$ and 40 wt.-% of $NaNO_2$ and heated to 180 °C. 1 mL of water has been added to the salt mixture obtaining the water-salt mixture. Then, the boiling point of the water fraction in the water-salt mixture has been determined at reflux to be 182 °C. No solid crystals have been determined under visual inspection at 182 °C.

Example 2

Next, the theoretical amount of water, or water steam, necessary to dissolve 100 g of a salt mixture comprising 60 wt.-% of $KNO_3$ and 40 wt.-% of $NaNO_2$ has been calculated. The aqueous solubility values of $KNO_3$ and $NaNO_2$ at a temperature of 20 °C are shown in table 1.

Table 1.

| salt | aqueous solubility at 20 °C [g/L] |
|------|-----------------------------------|
| $KNO_3$ | 316 |
| $NaNO_2$ | 820 |

As $KNO_3$ exhibits a lower aqueous solubility than $NaNO_2$, the content of $KNO_3$ in a mixture of $KNO_3$ and $NaNO_2$ determines the overall solubility of said mixture in water.

A saturated aqueous solution of $KNO_3$ exhibits a density of 1164 g/L. Thus, 1 L of said saturated aqueous solution of $KNO_3$ consists of 316 g of $KNO_3$ and 848 g of water. Consequently, the amount of water necessary for dissolving 100 g of the salt mixture comprising 60 wt.-% of $KNO_3$ and 40 wt.-% of $NaNO_2$ may be calculated as follows:

$$m \text{ (water)} = m \text{ } (KNO_3) \cdot 848 \text{ g} / 316 \text{ g} = 60 \text{ g} \cdot 848 / 316 = 161 \text{ g}$$

With these data in hand, the salt mixture comprising 60 wt.-% of $KNO_3$ and 40 wt.-% of $NaNO_2$ has been investigated for occurrence of solid crystals after heating the salt mixture to 182 °C, adding specific amounts of waters and cooling the resulting water-salt mixture from 182 °C to room temperature. Three different amounts of water have been added in the form of water steam to said salt mixture as shown in table 2.

Table 2.

| # | salt mixture (wt.-%) | amount of salt mixture [g] | amount of water steam [1] [g] | precipitate detected by visual inspection |
|---|----------------------|-----------------------------|-------------------------------|-------------------------------------------|
| 1 | $KNO_3$ / $NaNO_2$ (60 : 40) | 100 | 100 | yes [2] |

(continued)

| # | salt mixture (wt.-%) | amount of salt mixture [g] | amount of water steam [1] [g] | precipitate detected by visual inspection |
|---|---|---|---|---|
| 2 | $KNO_3$ / $NaNO_2$ (60 : 40) | 100 | 159 | no |
| 3 | $KNO_3$ / $NaNO_2$ (60 : 40) | 100 | 164 | no |
| [1] The exact amounts of water steam have been determined gravimetrically. [2] The precipitate has been determined to be $KNO_3$ by Immediate Constituent Analysis (ICA) and Elemental Analysis (EA). | | | | |

The results as shown in table 2 match with the above calculated substance data, i.e. the theoretical amount of water necessary for dissolving 100 g of the salt mixture comprising 60 wt.-% of $KNO_3$ and 40 wt.-% of $NaNO_2$ (see above, 161 g) upon cooling the water-salt mixture from 182 °C to room temperature.

The question raised at the beginning whether the above salt mixture may be mixed with water while controlling the water concentration such that the resulting water-salt mixture may be cooled down from 182 °C to room temperature without generating any solid salt crystals may be answered "yes".

The results obtained in examples 1 and 2 have been transferred to a large-scale industrial plant, i.e. a tubular reactor, for investigation in a shut-down process of said reactor. See example 3.

Example 3

The working example 3 described hereinafter relates to a tubular reactor 1 in which a heterogeneously catalyzed gas phase oxidation of acrolein 19 to acrylic acid 20 using oxygen of air is carried out. However, the process described herein shall not be limited to the above oxidation reaction. The reactor of the working example 3 comprises 28,000 reaction tubes 2 with an outer diameter of 30 mm and a tube length of 3200 mm. The volume on the salt side of the reactor sums up to about 110 $m^3$.

The tubular reactor 1 of the process of the present invention comprises a reactor shell 7 in the form of a cylindrical body which is sealed with an upper hood 3 on the upper end of the reactor shell 7 and a lower hood 4 at the lower end of the reactor shell 7, wherein the upper hood 3 and the lower hood 4 are connected to the reactor shell 7 in a gas-tight manner. Inside the tubular reactor 1, vertically arranged reaction tubes 2 are present in such a way that the reactor shell 7 encloses the reaction tubes 2. The upper ends of the reaction tubes 2 are connected to an upper tube sheet 5 and the lower ends of the reaction tubes 2 are connected to a lower tube sheet 6, each in a gas-tight manner. Further, the upper tube sheet 5 and the lower tube sheet 6 are connected to the tubular reactor 1, each in a gas-tight manner. Thus a gas-tight reaction space is formed inside the tubular reactor 1 between the upper hood 3 and the upper tube sheet 5, inside the reaction tubes 2, and the lower tube sheet 6 and the lower hood 4.

A liquid-tight heat transfer space 9 is formed inside the tubular reactor 1 between the upper tube sheet 5, the lower tube sheet 6, and outside the reaction tubes 2, i.e. the heat transfer space 9 is a separate space located outside of the above-described reaction space and inside of the reactor shell 7 of the tubular reactor 1.

The reaction tubes 2 are arranged in the tubular reactor 1 such that they are equidistant from each other. The reaction tubes 2 of the tubular reactor 1 comprise a granular molybdenum catalyst for carrying out the above-mentioned heterogeneously catalyzed gas phase oxidation of acrolein 19 to acrylic acid 20 using oxygen of air. Said granular molybdenum catalyst is a multimetal oxide catalyst comprising molybdenum and is present in the form of a fixed bed inside the reaction tubes 2. As such granular molybdenum catalysts are known per se, they are not described in further detail herein.

During operation mode, i.e. carrying out the above-mentioned gas phase oxidation, the temperature inside the tubular reactor 1 needs to be raised to a reaction temperature which is in the range of 265 to 300 °C. Said reaction temperature is provided using a heat transfer medium which is a eutectic salt mixture. Specifically, a substantially anhydrous liquefied salt melt 8 consisting of 55 wt.-% of $KNO_3$ and 45 wt.-% of $NaNO_2$ is used as heat transfer medium. Said liquefied salt melt 8 has a melting temperature of 142 °C and a solubility such that 1 kg of liquefied salt melt 8 is soluble in 1.55 L of water, at 20 °C. The liquefied salt melt 8 is heated to the above reaction temperature and then circulated through the heat transfer space 9 between the reaction tubes 2 and the reactor shell 7 in a longitudinal direction using a circulation pump 23 having a pump casing 31. By said circulation, stable reaction conditions, i.e. a constant temperature distribution throughout the whole volume of the tubular reactor 1 is ensured, especially at the reaction tubes 2 where the oxidation reaction occurs. The liquefied salt melt 8 is tempered to the reaction temperature using a heat exchanger 15 which is operated by water 10 to generate a water steam 17 at a pressure of 25 bar. In other words, the liquefied salt melt 8 is cooled in the heat exchanger 15 and transfers the reaction enthalpy generated in the reaction tubes 2 to the heat exchanger 15.

For carrying out the above-mentioned gas phase oxidation reaction of acrolein 19 to acrylic acid 20 during operation mode, the feed gas, i.e. acrolein 19, is introduced into the reaction space of the tubular reactor 1 via the inlet part of the tubular reactor 1 at the upper hood 3. Subsequently, acrolein 19 enters the reaction tubes 2 at the upper ends of the reaction tubes 2 via the upper tube sheet 5 and flows through the reaction tubes 2 in direction from the upper ends of the reaction tubes 2 towards the lower ends of the reaction tubes 2, i.e. in direction to the lower tube sheet 6. Inside the reaction tubes 2, acrolein 19 is subjected to the heterogeneously catalyzed gas phase oxidation reaction at granular molybdenum catalyst using oxygen of air and recycle gas as diluent yielding acrylic acid 20. Acrylic acid 20 leaves the reaction tubes 2 at their lower ends via the lower tube sheet 6 and is then discharged at the bottom part of the tubular reactor 1 via the lower hood 4.

The above-described granular molybdenum catalyst, which is present inside the reaction tubes 2 of the tubular reactor 1, has a certain lifetime which is in the range of several years. After exceeding its lifetime, the spent granular molybdenum catalyst is no longer active in the oxidation reaction. Thus, the spent granular molybdenum catalyst has to be removed from the reaction tubes 2 and replaced with new granular molybdenum catalyst. This process is referred to as catalyst replacement shut-down. During this shut-down, the operation mode of the tubular reactor 1 is interrupted as it has to be opened in order to be able to empty and refill the reaction tubes 2. This means that the pressure and the temperature of the tubular reactor 1 need to be reduced. In the context of the present working example 3, the tubular reactor 1 is brought to ambient pressure and room temperature.

The inventive shut-down process for cooling the tubular reactor 1 from the reaction temperature to room temperature is described in the following: Prior to cooling the tubular reactor 1 from the reaction temperature to room temperature, the gas flow of acrolein 19 is interrupted, a water steam outlet 18 of the heat exchanger 15 is routed to ambient and water 10 in the form of boiler feed water is fed to the heat exchanger 15 and forms the water steam 17 at almost ambient pressure.

In a first phase of cooling down, the temperature of the tubular reactor 1 is reduced from the reaction temperature of 295 °C to about 180 °C by evaporation of water 10 in the form of boiler feed water in the heat exchanger 15.

In a second phase of cooling down, i.e. further cooling from about 180 °C, additional demineralized water 24 is slowly fed to the suction side of the circulation pump 23 via a demineralized water supply 30, i.e., demineralized water 24 is fed into the heat transfer space 9 of the tubular reactor 1, obtaining a water-salt mixture 11. At the beginning of the second phase, minor amounts of a water steam 21 and a water steam 26 are generated which are released from the system via a vent line 22 at the heat exchanger 15 and a vent line 25 at the pump casing 31, respectively.

In a third phase of cooling down, as soon as almost no water steam 21 is vented via the vent line 22 and almost no water steam 26 is vented via the vent line 25, the demineralized water supply 30 is fully opened, allowing for more demineralized water 24 flowing into the heat-transfer space 9 of the tubular reactor 1. Furthermore, a water tube 14 to the heat exchanger 15 is switched to cold demineralized water for further cooling.

By the addition of the demineralized water 24 via the demineralized water supply 30, the volume of the water-salt mixture 11 increases inside the tubular reactor 1. Exceeding water-salt mixture 27 overflows via an overflow line 28 of the pump casing 31 and is routed to a reservoir 12.

Samples are taken from the exceeding water-salt mixture 27 at the overflow line 28 during cooling down and the demineralized water supply 30 is stopped as soon as the density of the exceeding water-salt mixture 27 at the overflow line 28 is < 1240 g/L at a temperature of 20 °C. Cooling via the heat exchanger 15 is stopped as soon as the temperature of the water-salt mixture 11 is < 40 °C. During the cooling down procedure and further on, the circulation pump 23 is kept running.

During the whole cooling step of the tubular reactor 1, the water-salt mixture 11 is kept in a liquefied state. Keeping the water-salt mixture 11 in a liquefied state does not require heating the water-salt mixture 11 as it is liquid even at room temperature. Thus, the water-salt mixture 11 does not need to be heated at any time during the shut-down process of the tubular reactor 1.

Generally, a first part of the water-salt mixture 11 is kept inside the tubular reactor 1, wherein a second part of the water-salt mixture 11 is stored outside the tubular reactor 1. This is achieved by discharging the second part of the water-salt mixture 11 into the external reservoir 12 via a non-heated drain tube 13 or via the overflow line 28. The reservoir 12 is an already existing salt melt reservoir which does not need to be heated and has a volume which is sufficient to take up the second part of the water-salt mixture 11. During cooling down, about 120 m$^3$ of the water-salt mixture 11 is released.

After interruption of the operation mode and cooling the tubular reactor 1 from the reaction temperature of 295 °C to 40 °C, the tubular reactor 1 is opened and the granular molybdenum catalyst is replaced from the reaction tubes 2. During the catalyst replacement, the temperature of the reactor dropped from 40 °C down to room temperature.

For returning to operation mode, a heating up step has to be performed after the catalyst replacement has been finished and the tubular reactor 1 has been closed resulting in that the operation mode is no longer interrupted.

The temperature of the tubular reactor 1 is increased by supplying heat via the heat exchanger 15 during the heating up step of the tubular reactor 1. The heat exchanger 15 is supplied with water steam at a pressure of 25 bar as reverse flow via the water steam outlet 18 during normal operation. The water steam condenses in the heat exchanger 15. Condensate is released from the heat exchanger 15 via a blow down line to ambient (not depicted).

Starting at room temperature, the temperature is successively raised to 190 °C. After reaching a temperature of about 120 °C, water present in the water-salt mixture 11 starts boiling. The water boiling out of the water-salt mixture 11 is released from the tubular reactor 1 in the form of the water steam 21 via the vent line 22 and water steam 26 via the vent line 25.

This results in a continuous removal of water 10 in the form of the water steam 21 and the water steam 26 from the water-salt mixture 11 during the heating up step and a volume reduction of the water-salt mixture 11. While continuously removing water 10 from the water-salt mixture 11, the original composition of the substantially anhydrous liquefied salt melt 8 of before the shut-down of the tubular reactor 1 is regained.

The volume reduction of the water-salt mixture 11 is compensated by successively adding the water-salt mixture 11 which is stored outside the tubular reactor 1 in the reservoir 12, i.e. the above-described second part of the water-salt mixture 11. Practically, after a certain volume of water 10 has been boiled out of the water-salt mixture 11, a similar volume of the externally stored water-salt mixture 11, i.e. the second part of the water-salt mixture 11, is pumped back into the tubular reactor 1 from the reservoir 12 via the drain tube 13 using a pump. Afterwards, another volume of water 10 is boiled out and the preceding steps are repeated.

During the removal of water 10 from the water-salt mixture 11, the temperature is continuously raised until a temperature of 190 °C is regained. After reaching 190 °C, the further heating up is supported by starting an electrical heater 29 of the tubular reactor 1 until the reaction temperature of 295 °C is reached. Only after said reaction temperature has been reached, the oxidation reaction of acrolein 19 to acrylic acid 20 may be continued by resuming the feeding of acrolein 19.

Noteworthy, the water-salt mixture 11 which is kept inside the tubular reactor 1, i.e. the above-described first part of the water-salt mixture 11, is continuously circulated through the tubular reactor 1 during the whole shut-down process using a pump. This leads to the fact that no limitations occur regarding heating rate and/or cooling rate of the water-salt mixture 11, and consequently of the tubular reactor 1 itself. In other words, the tubular reactor 1 can be cooled as quickly as possible in the context of the present working example 3.

The time demand for cooling down and heating up in the working example 3 is illustrated in the following:

Phase 1: cooling down from reaction temperature to 180 °C with boiler feed water: 4 h.

Phase 2: cooling down from 180 °C to 40 °C by feeding additional water to the unit: 26 h.

Phase 3: heating up from room temperature to 190 °C by steam of the heat exchanger 15: 29 h.

Phase 4: heating up from 190 °C to 265 °C by electrical heater: 19 h.

Total time demand for cooling down and heating up: 78 h.

Comparative example

The reactor unit of example 3 was cooled down and heated up applying the method described in EP 1 882 518 B1 by feeding gas at various temperatures and safeguarding maximum temperature differences across the whole equipment. By applying this method, the following time demands are necessary:

Phase 1: cooling down from reaction temperature to 180 °C with boiler feed water: 4 h.

Phase 2: release of the completed salt melt to a salt melt drum: 23 h.

Phase 3: cooling down from 180 °C to 40 °C by feeding gas with decreasing temperature through the reaction tubes: 36 h.

Phase 4: heating up from room temperature to 190 °C with gas with increasing temperature: 72 h.

Phase 5: filling with salt from the salt melt drum: 6 h.

Phase 5: heating up of the salt melt from 190 °C to the reaction temperature: 17 h.

Total time demand for cooling down and heating up: 158 h.

List of reference signs

1 tubular reactor
2 reaction tube
3 upper hood
4 lower hood
5 upper tube sheet
6 lower tube sheet
7 reactor shell
8 liquefied salt melt
9 heat transfer space
10 water
11 water-salt mixture
12 reservoir
13 drain tube
14 water tube
15 heat exchanger
16 heat exchanger tube17 water steam
18 water steam outlet
19 acrolein
20 acrylic acid
21 water steam
22 vent line
23 circulation pump
24 demineralized water
25 vent line
26 water steam
27 exceeding water-salt mixture
28 overflow line
29 electrical heater
30 demineralized water supply
31 pump casing

**Claims**

1. A process for shutting-down a tubular reactor (1) for a catalytic gas phase reaction from a reaction temperature, wherein the tubular reactor (1) comprises

    - a plurality of vertically arranged reaction tubes (2),
    - an upper tube sheet (5) and a lower tube sheet (6) which each are connected to upper ends and lower ends of the reaction tubes (2) in a gas-tight manner, and
    - a reactor shell (7) which encloses the plurality of reaction tubes (2) forming a liquid-tight heat transfer space (9), wherein, in operation mode, a substantially anhydrous liquefied salt melt (8) is circulated in the heat transfer space (9),
    **characterized in that**

    - water (10) is added to the substantially anhydrous liquefied salt melt (8), obtaining a water-salt mixture (11), while cooling the tubular reactor (1) to a temperature below the solidification temperature of the substantially anhydrous liquefied salt melt (8), such that
    - the water-salt mixture (11) is kept in a liquefied state during the whole cooling step of the tubular reactor (1).

2. The process according to claim 1, wherein the water-salt mixture (11) is cooled to a temperature in the range of 80 to 10 °C, preferably 60 to 20 °C, more preferably to room temperature.

3. The process according to claim 1 or 2, wherein the water (10) is added to obtain a weight ratio of water (10) to liquefied salt melt (8) of 80 : 20 to 40 : 60, preferably 60 : 40 to 40 : 60.

4. The process according to any one of the preceding claims, wherein the liquefied salt melt (8) has a melting temperature in the range of 100 to 450 °C, preferably 140 to 155 °C.

5. The process according to any one of the preceding claims, wherein the liquefied salt melt (8) has a solubility such that 1 kg of liquefied salt melt (8) is soluble in less than 6 L, preferably less than 3 L of water (10), at 20 °C.

6. The process according to any one of the preceding claims, wherein the liquefied salt melt (8) is an eutectic mixture comprising nitrate moieties, preferably a mixture of at least two salts selected from alkali nitrates, alkali nitrites and alkali carbonates, preferably a mixture of two or three of the salts potassium nitrate, sodium nitrate and sodium nitrite.

7. The process according to any one of the preceding claims, wherein the liquefied salt melt (8) consists of a mixture of the salts potassium nitrate and sodium nitrite in a weight ratio of potassium nitrate to sodium nitrite of 45 : 65 to 65 : 45, preferably 50 : 50 to 60 : 40.

8. The process according to any one of the preceding claims, wherein at least a part of the water-salt mixture (11) is kept inside the tubular reactor (1).

9. The process according to claim 8, wherein the water-salt mixture (11) which is kept inside the tubular reactor (1) is continuously circulated through the tubular reactor (1).

10. The process according to any one of the preceding claims, wherein at least a part of the water-salt mixture (11) is stored outside the tubular reactor (1), preferably the water-salt mixture (11) is introduced into a non-heated reservoir (12) via a drain tube (13).

11. A process for heating up the tubular reactor (1) from the temperature below the solidification temperature of the liquefied salt melt (8) or lower to the reaction temperature after the cooling step according to any one of claims 1 to 10, wherein at least a part of the water (10) is boiled out from the water-salt mixture (11) at a temperature above the solidification temperature of the liquefied salt melt (8) by supplying heat via a heat exchanger (15) obtaining a water steam (21) and resulting in a volume reduction of the water-salt mixture (11).

12. The process according to claim 11, wherein the water steam (21) is released from the tubular reactor (1), preferably via a vent line (22).

13. The process according to any one of claims 11 or 12, wherein the volume reduction of the water-salt mixture (11) is compensated by adding at least a part of the water-salt mixture (11) which is stored outside the tubular reactor (1).

14. The process according to any one of the preceding claims, wherein the tubular reactor (1) is cooled down, a catalyst which is present inside the reaction tubes (2) is replaced, and the tubular reactor (1) is then heated up.

15. The process according to any one of the preceding claims, wherein the gas phase reaction is selected from oxidation, hydrogenation, dehydrogenation, nitration, and alkylation reactions, preferably an oxidation reaction, in particular an oxidation reaction of acrolein (19) to acrylic acid (20).

**Patentansprüche**

1. Verfahren zum Abschalten eines Rohrreaktors (1) für eine katalytische Gasphasenreaktion ausgehend von einer Reaktionstemperatur, wobei der Rohrreaktor (1) Folgendes umfasst:

   - eine Mehrzahl vertikal angeordneter Reaktionsrohre (2),
   - einen oberen Rohrboden (5) und einen unteren Rohrboden (6), die jeweils gasdicht mit oberen Enden und unteren Enden der Reaktionsrohre (2) verbunden sind, und
   - eine Reaktorhülle (7), welche die Mehrzahl von Reaktionsrohren (2) umschließt, wodurch ein flüssigkeitsdichter Wärmeübertragungsraum (9) ausgebildet wird,

   wobei im Betriebsmodus eine im Wesentlichen wasserfreie verflüssigte Salzschmelze (8) in dem Wärmeübertragungsraum (9) im Kreis geführt wird,
   **dadurch gekennzeichnet, dass**

- Wasser (10) zu der im Wesentlichen wasserfreien verflüssigten Salzschmelze (8) zugegeben wird, wodurch ein Wasser-Salz-Gemisch (11) erhalten wird, während der Rohrreaktor (1) auf eine Temperatur unter der Erstarrungstemperatur der im Wesentlichen wasserfreien verflüssigten Salzschmelze (8) abgekühlt wird, derart dass
- das Wasser-Salz-Gemisch (11) während des gesamten Abkühlungsschritts des Rohrreaktors (1) in einem verflüssigten Zustand gehalten wird.

2. Verfahren nach Anspruch 1, wobei das Wasser-Salz-Gemisch (11) auf eine Temperatur im Bereich von 80 bis 10 °C, vorzugsweise 60 bis 20 °C, besonders bevorzugt auf Raumtemperatur, abgekühlt wird.

3. Verfahren nach Anspruch 1 oder 2, wobei das Wasser (10) zugegeben wird, um ein Gewichtsverhältnis von Wasser (10) zu verflüssigter Salzschmelze (8) von 80 : 20 bis 40 : 60, vorzugsweise 60 : 40 bis 40 : 60, zu erhalten.

4. Verfahren nach einem der vorangehenden Ansprüche, wobei die verflüssigte Salzschmelze (8) eine Schmelztemperatur im Bereich von 100 bis 450 °C, vorzugsweise 140 bis 155 °C, aufweist.

5. Verfahren nach einem der vorangehenden Ansprüche, wobei die verflüssigte Salzschmelze (8) eine Löslichkeit derart aufweist, dass 1 kg verflüssigte Salzschmelze (8) bei 20 °C in weniger als 6 l, vorzugsweise weniger als 3 l, Wasser (10) löslich ist.

6. Verfahren nach einem der vorangehenden Ansprüche, wobei die verflüssigte Salzschmelze (8) ein eutektisches Gemisch ist, das Nitratkomponenten umfasst, vorzugsweise ein Gemisch von wenigstens zwei Salzen, ausgewählt aus Alkalinitraten, Alkalinitriten und Alkalicarbonaten, vorzugsweise ein Gemisch von zwei oder drei der Salze Kaliumnitrat, Natriumnitrat und Natriumnitrit.

7. Verfahren nach einem der vorangehenden Ansprüche, wobei die verflüssigte Salzschmelze (8) aus einem Gemisch der Salze Kaliumnitrat und Natriumnitrit in einem Gewichtsverhältnis von Kaliumnitrat zu Natriumnitrit von 45 : 65 bis 65 : 45, vorzugsweise 50 : 50 bis 60 : 40, besteht.

8. Verfahren nach einem der vorangehenden Ansprüche, wobei wenigstens ein Teil des Wasser-Salz-Gemischs (11) im Inneren des Rohrreaktors (1) gehalten wird.

9. Verfahren nach Anspruch 8, wobei das Wasser-Salz-Gemisch (11), das im Inneren des Rohrreaktors (1) gehalten wird, kontinuierlich durch den Rohrreaktor (1) im Kreis geführt wird.

10. Verfahren nach einem der vorangehenden Ansprüche, wobei wenigstens ein Teil des Wasser-Salz-Gemischs (11) außerhalb des Rohrreaktors (1) gespeichert wird, vorzugsweise das Wasser-Salz-Gemisch (11) über ein Ablassrohr (13) in ein unbeheiztes Reservoir (12) eingebracht wird.

11. Verfahren zum Aufheizen des Rohrreaktors (1) von der Temperatur unter der Erstarrungstemperatur der verflüssigten Salzschmelze (8) oder darunter auf die Reaktionstemperatur nach dem Abkühlungsschritt nach einem der Ansprüche 1 bis 10, wobei wenigstens ein Teil des Wassers (10) aus dem Wasser-Salz-Gemisch (11) bei einer Temperatur über der Erstarrungstemperatur der verflüssigten Salzschmelze (8) ausgekocht wird, indem über einen Wärmetauscher (15) Wärme zugeführt wird, wodurch ein Wasserdampf (21) erhalten wird und was eine Volumenreduktion des Wasser-Salz-Gemischs (11) zur Folge hat.

12. Verfahren nach Anspruch 11, wobei der Wasserdampf (21) aus dem Rohrreaktor (1) heraus freigesetzt wird, vorzugsweise über eine Entlüftungsleitung (22).

13. Verfahren nach einem der Ansprüche 11 oder 12, wobei die Volumenreduktion des Wasser-Salz-Gemischs (11) ausgeglichen wird, indem wenigstens ein Teil des Wasser-Salz-Gemischs (11), das außerhalb des Rohrreaktors (1) gespeichert ist, zugegeben wird.

14. Verfahren nach einem der vorangehenden Ansprüche, wobei der Rohrreaktor (1) abgekühlt wird, ein Katalysator, der im Inneren der Reaktionsrohre (2) vorhanden ist, ausgetauscht wird und der Rohrreaktor (1) dann aufgeheizt wird.

15. Verfahren nach einem der vorangehenden Ansprüche, wobei die Gasphasenreaktion aus Oxidations-, Hydrierungs-, Dehydrierungs-, Nitrierungs- und Alkylierungsreaktionen, vorzugsweise einer Oxidationsreaktion, insbesondere einer Oxidationsreaktion von Acrolein (19) zu Acrylsäure (20), ausgewählt ist.

**Revendications**

1. Processus d'arrêt d'un réacteur tubulaire (1) pour une réaction en phase gazeuse catalytique à partir d'une température de réaction, le réacteur tubulaire (1) comprenant

   - une pluralité de tubes de réaction (2) agencés verticalement,
   - une feuille de tube supérieure (5) et une feuille de tube inférieure (6) qui sont chacune reliées à des extrémités supérieures et à des extrémités inférieures des tubes de réaction (2) de manière étanche aux gaz, et
   - une enveloppe de réacteur (7) qui renferme la pluralité de tubes de réaction (2) formant un espace de transfert de chaleur étanche aux liquides (9),

   dans lequel, en mode fonctionnement, une masse fondue de sel liquéfié substantiellement anhydre (8) est mise en circulation dans l'espace de transfert de chaleur (9),
   **caractérisé en ce que**

   - de l'eau (10) est ajoutée à la masse fondue de sel liquéfié substantiellement anhydre (8), obtenant un mélange eau-sel (11), tout en refroidissant le réacteur tubulaire (1) à une température au-dessous de la température de solidification de la masse fondue de sel liquéfié substantiellement anhydre (8), de telle sorte que
   - le mélange eau-sel (11) soit maintenu dans un état liquéfié pendant toute l'étape de refroidissement du réacteur tubulaire (1).

2. Procédé selon la revendication 1, dans lequel le mélange eau-sel (11) est refroidi à une température dans la plage allant de 80 à 10 °C, préférablement de 60 à 20 °C, plus préférablement à température ambiante.

3. Procédé selon la revendication 1 ou la revendication 2, dans lequel l'eau (10) est ajoutée pour obtenir un rapport pondéral de l'eau (10) à la masse fondue de sel liquéfié (8) allant de 80/ 20 à 40/ 60, préférablement de 60/ 40 à 40/ 60.

4. Procédé selon l'une quelconque des revendications précédentes, dans lequel la masse fondue de sel liquéfié (8) a une température de fusion dans la plage allant de 100 à 450 °C, préférablement de 140 à 155 °C.

5. Procédé selon l'une quelconque des revendications précédentes, dans lequel la masse fondue de sel liquéfié (8) a une solubilité telle que 1 kg de masse fondue de sel liquéfié (8) soit soluble dans moins de 6 L, préférablement moins de 3 L d'eau (10), à 20 °C.

6. Procédé selon l'une quelconque des revendications précédentes, dans lequel la masse fondue de sel liquéfié (8) est un mélange eutectique comprenant des fractions de nitrate, préférablement un mélange d'au moins deux sels sélectionnés parmi les nitrates alcalins, les nitrites alcalins et les carbonates alcalins, préférablement un mélange de deux ou trois sels parmi les sels de nitrate de potassium, de nitrate de sodium et de nitrite de sodium.

7. Procédé selon l'une quelconque des revendications précédentes, dans lequel la masse fondue de sel liquéfié (8) est constituée d'un mélange des sels de nitrate de potassium et de nitrite de sodium dans un rapport pondéral nitrate de potassium/nitrite de sodium allant de 45/ 65 à 65/ 45, préférablement de 50/ 50 à 60/ 40.

8. Procédé selon l'une quelconque des revendications précédentes, dans lequel au moins une partie du mélange eau-sel (11) est maintenue à l'intérieur du réacteur tubulaire (1).

9. Procédé selon la revendication 8, dans lequel le mélange eau-sel (11) qui est maintenu à l'intérieur du réacteur tubulaire (1) est mis en circulation en continu à travers le réacteur tubulaire (1).

10. Procédé selon l'une quelconque des revendications précédentes, dans lequel au moins une partie du mélange eau-sel (11) est stockée à l'extérieur du réacteur tubulaire (1), préférablement le mélange eau-sel (11) est introduit dans un réservoir non chauffé (12) par l'intermédiaire d'un tube de drainage (13).

11. Procédé de chauffage du réacteur tubulaire (1) à partir de la température au-dessous de la température de solidification de la masse fondue de sel liquéfié (8) ou inférieure à la température de réaction après l'étape de refroidissement selon l'une quelconque des revendications 1 à 10, dans lequel au moins une partie de l'eau (10) est évaporée par ébullition hors du mélange eau-sel (11) à une température au-dessus de la température de solidification de la masse fondue de sel liquéfié (8) en fournissant de la chaleur par l'intermédiaire d'un échangeur de chaleur (15) obtenant une vapeur d'eau (21) et conduisant à une réduction de volume du mélange eau-sel (11).

12. Procédé selon la revendication 11, dans lequel la vapeur d'eau (21) est libérée du réacteur tubulaire (1), préférablement par l'intermédiaire d'une conduite d'évent (22).

13. Procédé selon l'une quelconque des revendications 11 ou 12, dans lequel la réduction de volume du mélange eau-sel (11) est compensée par l'ajout d'au moins une partie du mélange eau-sel (11) qui est stocké à l'extérieur du réacteur tubulaire (1).

14. Procédé selon l'une quelconque des revendications précédentes, dans lequel le réacteur tubulaire (1) est refroidi, un catalyseur qui est présent à l'intérieur des tubes de réaction (2) est remplacé, et le réacteur tubulaire (1) est ensuite chauffé.

15. Procédé selon l'une quelconque des revendications précédentes, dans lequel la réaction en phase gazeuse est sélectionnée parmi des réactions d'oxydation, d'hydrogénation, de déshydrogénation, de nitration, et d'alkylation, préférablement une réaction d'oxydation, en particulier une réaction d'oxydation de l'acroléine (19) en acide acrylique (20).

FIG.1

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1882518 B1
- DE 102014103691 A1